# EUROPEAN PATENT SPECIFICATION

(11) **EP 1 797 921 B1**
(45) Date of publication and mention of the grant of the patent: **23.12.2009**
(21) Application number: 06017006.5
(22) Date of filing: 16.08.2006
(51) Int. Cl.: A61M 5/50, A61M 5/32, A61M 5/34, A61M 5/315

(54) **Safety hypodermic syringe**
Sicherheits-Subkutaninjektions-spritze
Seringue hypodermique de sécurité

(30) Priority: 19.12.2005 CN 200510134691
(43) Date of publication of application: 20.06.2007
(73) Proprietor: Chen, Chang-Tzu, Taipei (TW)
(72) Inventor: Chen, Chang-Tzu, Taipei (TW)
(74) Representative: Viering, Jentschura & Partner

(56) References cited:
- EP-A2- 0 590 757
- US-A- 5 458 576
- US-A1- 2004 122 364
- US-A1- 2004 236 283
- US-A1- 2005 107 747
- US-B1- 6 432 082

## Description

### BACKGROUND OF THE INVENTION

### Field of the Invention:

The present invention relates to a safety hypodermic syringe and more particularly, to such a safety hypodermic syringe that prevents breaking of the breakable front wall of the barrel accidentally during transportation.

### Description of the Related Art:

A hypodermic syringe is an important medical instrument adapted for use to inject or withdraw liquid medicines. In recent years, the spreading of AIDS and many other diseases that are difficult to treat causes many contamination accidents. In order to prevent contamination, safety hypodermic syringes are developed. A safety hypodermic syringe is known comprising a needle assembly, a medicine barrel, a plunger, and an outer barrel. The needle assembly is fastened to the front side of the medicine barrel. The plunger is axially movably coupled to the medicine barrel and mounted with the medicine barrel inside the outer barrel. After the service of the hypodermic syringe, the plunger is pulled backwards to carry the needle cannula or the needle assembly backwards to the inside of the medicine barrel. This design of safety hypodermic syringe is functional, however it has a complicated structure and comprised of a big number of parts, resulting in a high manufacturing cost.

Taiwan Patent Publication No. 540385, issued on July 1^{st}, 2003, discloses a safety hypodermic syringe entitled "Two Piece Type Retractable Safety Hypodermic Syringe". According to this design, the safety hypodermic syringe comprises a barrel, a plunger inserted into the barrel, a needle holder provided at the front side of the barrel to hold a needle, and a block with a through hole installed in the needle holder. The barrel has a tear groove on the front wall around the needle holder. After engagement of the head of the plunger with the needle holder, the plunger is pulled backwards to break the tear groove, causing the needle holder and the needle to be carried with the plunger backwards to the inside of the barrel. According to this design, the thin tear groove on the front wall of the barrel may be broken accidentally during injection or transportation of the safety hypodermic syringe, thereby causing a leakage of the fluid medicine.

A further example of prior art safety hypodermic syringe is given in US 2004/122364 A1.

Further, according to conventional designs, a residual amount of the applied liquid medicine will be left in the tubular front neck of the barrel and the hub of the needle assembly after the stopper has been pushed to the front limit position and stopped at the front wall of the barrel.

Therefore, it is desirable to provide a safety hypodermic syringe that eliminates the aforesaid drawbacks.

### SUMMARY OF THE INVENTION

The present invention has been accomplished under the circumstances in view. It is the main object of the present invention to provide a safety hypodermic syringe, which is self-destructive, preventing reuse of the syringe. It is another object of the present invention to provide a safety hypodermic syringe, which expels liquid medicine completely out of the syringe during the injection, preventing residual liquid medicine in the barrel. It is still another object of the present invention to provide a safety hypodermic syringe, which keeps the needle assembly firmly secured to the inside of the barrel, preventing possible contamination.

According to one embodiment of the present invention, the safety hypodermic syringe comprises safety hypodermic syringe comprises a barrel, which comprises a fluid, a front wall, a tubular neck forwardly and perpendicularly extending from the front wall and defining a fluid passage in communication with the fluid chamber, a race forwardly and perpendicularly extending from the front wall around the tubular neck, a spiral tear groove formed on the front wall 1 adjacent to the race, and annular retaining rib extending around the inside wall of the tubular neck adjacent to the front wall, a needle assembly, which comprises a hub fitted onto the tubular neck of the barrel and a needle cannula forwardly extending from the hub, a plunger, which comprises a shank, a head at the front side of the shank, a front extension rod axially forwardly extending from the head, a conical front tip, and a front neck axially connected between the front extension rod and the conical front tip, a flexible stopper, which fits the diameter of the fluid chamber of the barrel and has a longitudinal open chamber axially extending to the rear side thereof and a front center through hole forwardly extending from the longitudinal chamber to the front side thereof for the passing of the front extension rod of the plunger, and a flexible support ring, which is sleeved onto the tubular neck and stopped against the front wall of the barrel between the tubular neck and the race. When pushed the plunger forwards after the service of the safety hypodermic syringe, the spiral tear groove is broken to separate the front wall from the barrel for enabling the broken front wall and the needle assembly to be pulled with the plunger to the inside of the barrel upon a return stroke of the plunger at this time.

According to another aspect of the present invention, the barrel has an inside annular stop flange extending around the inside wall near the rear end thereof. The head of the plunger has a front flange and a rear flange for engagement with the inside annular stop flange of the barrel after a return stroke of the plunger after the service of the safety hypodermic syringe.

According to still another aspect of the present invention, the plunger further has a rear neck connected between the shank and the head for breaking to separate the shank from the head after engagement of the front flange and rear flange of the head with the inside annular stop flange of the barrel.

According to still another aspect of the present invention, the shank of said plunger is radially ribbed, having a thumb rear at a rear end thereof. The plunger has a stopper holder formed of a parachute-like front skirt and a parachute-like rear skirt around the periphery of the front extension rod for holding the stopper. The topper has an inside annular flange formed in the rear side of the longitudinal chamber and engaged in between the front skit and rear skirt of the stopper holder of the plunger.

According to still another aspect of the present invention, the plunger further comprises a thumb rest at the rear end of the shank and a protruding strip forwardly extending from the thumb rest for stopping against the finger flange at the rear end of the barrel before the service of the safety hypodermic syringe. The protruding strip has a breakable reduced rear end connected to the thumb rest.

According to still another aspect of the present invention, the front extension rod of the plunger has a sloping face adapted to guide backward movement of the broken front wall to the inside the barrel to bias the needle assembly in the fluid chamber of the barrel after a return stroke of the plunger after the service of the safety hypodermic syringe.

According to still another aspect of the present invention, the support ring is made out of an elastic material.

### BRIEF DESCRIPTION OF THE DRAWINGS

FIG. 1a is an exploded view of a safety hypodermic syringe according to a first embodiment of the present invention.
FIG. 1b corresponds to FIG. 1a when viewed from another angle.
FIG. 2 is a perspective assembly view of the safety hypodermic syringe according to the present invention.
FIG. 3a is a top view in an enlarged scale of the safety hypodermic syringe according to the present invention.
FIG. 3b is a sectional front view in an enlarged scale of the front part of the barrel of the safety hypodermic syringe according to the present invention.
FIG. 4a is a sectional view of the present invention, showing a standby status of the safety hypodermic syringe.
FIG. 4b is a schematic drawing of a part of the present invention, showing the protruding strip of the plunger biased.
FIG. 5 is a schematic sectional view of the present invention, showing the barrel of the safety hypodermic syringe filled with a liquid medicine for injection.
FIG. 6 is a schematic sectional view of the present invention, showing the status of the hypodermic syringe after injection.
FIG. 7 is a schematic sectional enlarged view of a part of the present invention, showing the conical front tip of the plunger inserted into the fluid passage and stopped at the front side of the annular retaining rib of the barrel.
FIG. 8 is a schematic sectional enlarged view of a part of the present invention, showing the front wall of the barrel broken.
FIG. 9 is a schematic sectional enlarged view of the present invention, showing the needle assembly received inside the barrel after return stroke of the plunger.
FIG. 10 is a schematic sectional enlarged view of the present invention, showing the second neck of the plunger broken, the needle assembly with the front part of the plunger left inside the barrel.

### DETAILED DESCRIPTION OF THE PREFERRED EMBODIMENT

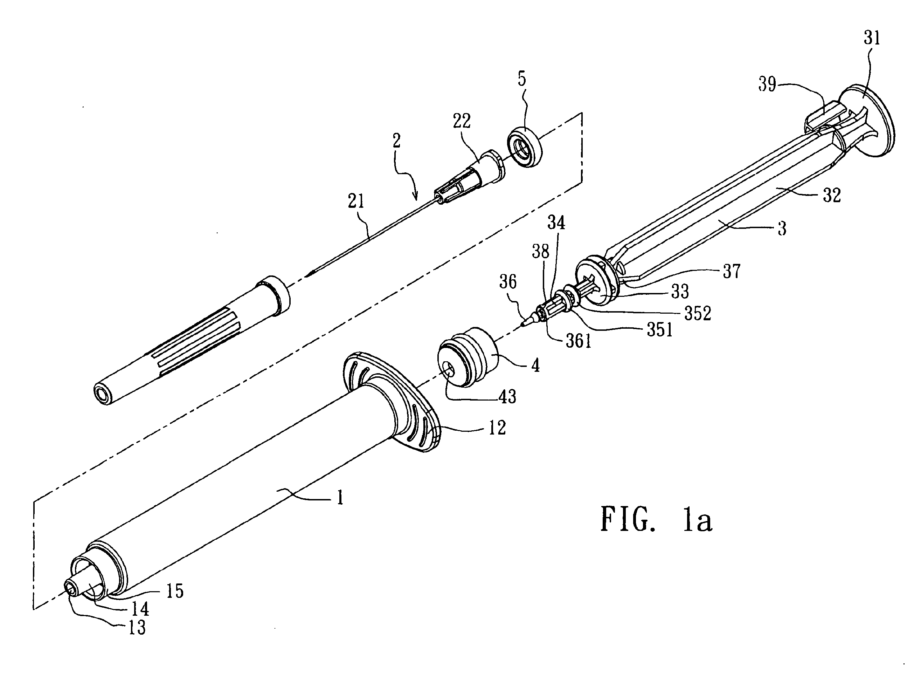

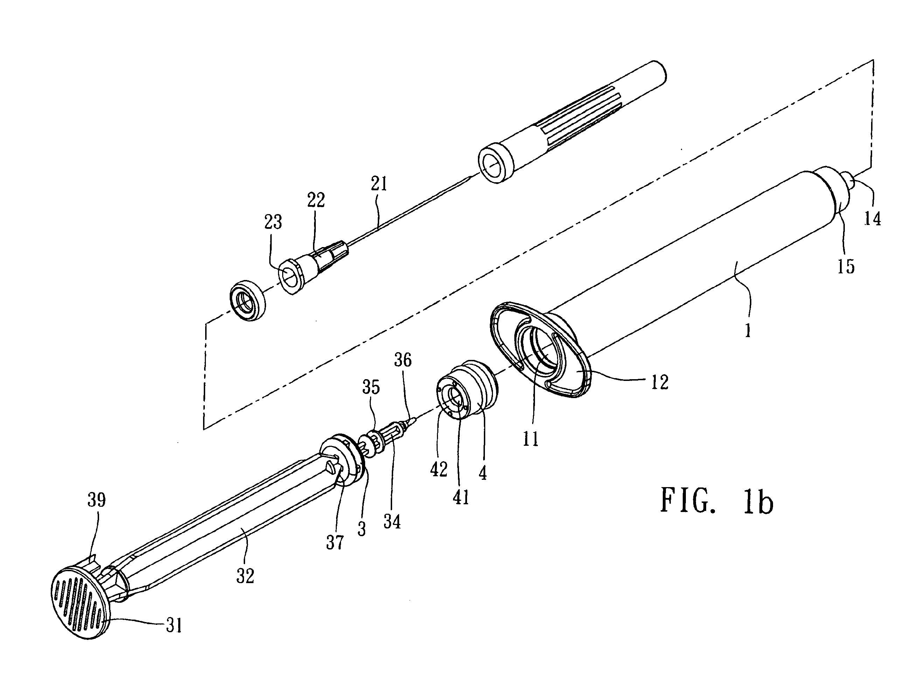

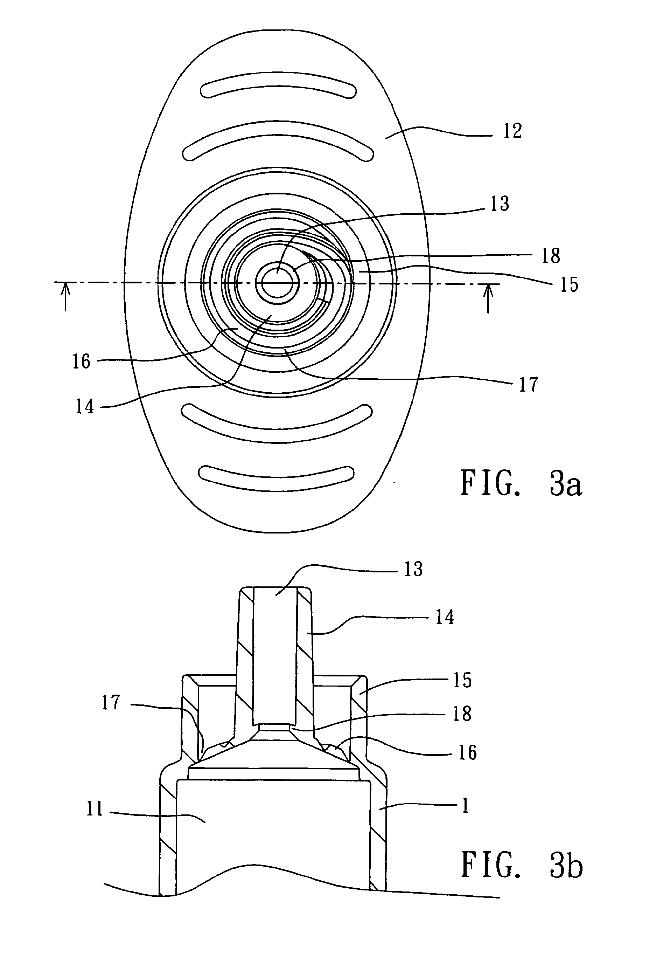

Referring to FIGS. 1~4, a safety hypodermic syringe in accordance with the present invention is shown comprised of a barrel **1,** a needle assembly **2,** a plunger **3,** a stopper **4,** and a support ring **5.**

The barrel **1** comprises a fluid chamber **11** adapted to hold a liquid medicine, a finger flange **12** extending around the periphery of the rear end thereof, a front wall **16,** a tubular neck **14** forwardly and perpendicularly extending from the front wall **16** and defining a fluid passage **13** in communication between the fluid chamber **11** and the atmosphere, a race **15** forwardly and perpendicularly extending from the front wall **16** around the tubular neck **14,** a spiral tear groove **17** formed on the front wall **16** adjacent to the race **15,** an annular retaining rib **18** extending around the inside wall of the tubular neck **14** adjacent to the front wall **16,** and an inside annular stop flange **19** extending around the inside wall corresponding to the finger flange **12.**

By means of the spiral tear groove **17,** the front wall **16** can easily be broken when the user pushes the plunger **3** with force, allowing the tubular neck **14** and the needle assembly **2** to be carried backwards into the inside of the barrel **1** by the plunger **3** after the service of the hypodermic syringe.

The support ring **5** is sleeved onto the tubular neck **14** and stopped against the front wall **16** of the barrel **1** between the tubular neck **14** and the race **15.** The support ring **5** can be made out of a hard or flexible material. Preferably, the support ring **5** is made out of rubber. The support ring **5** has two functions. The first function of the support ring **5** is to support the front wall **16,** presenting breaking of the tear groove **17** by a torque when the tubular neck **14** receives an external pressure accidentally. The second function of the support ring **5** is to prevent leakage of the contained liquid medicine out of the fluid chamber **11** when the tear groove **17** is broken accidentally.

The needle assembly **2** comprises a hub **22** and a needle cannula **21** fastened to the hub **22.** The hub **22** has a mounting hole **23** fitting the periphery of the tubular neck **14** and in fluid communication with the axial center through hole of the needle cannula **21.** Preferably, the tubular neck **14** and the mounting hole **23** are tapered.

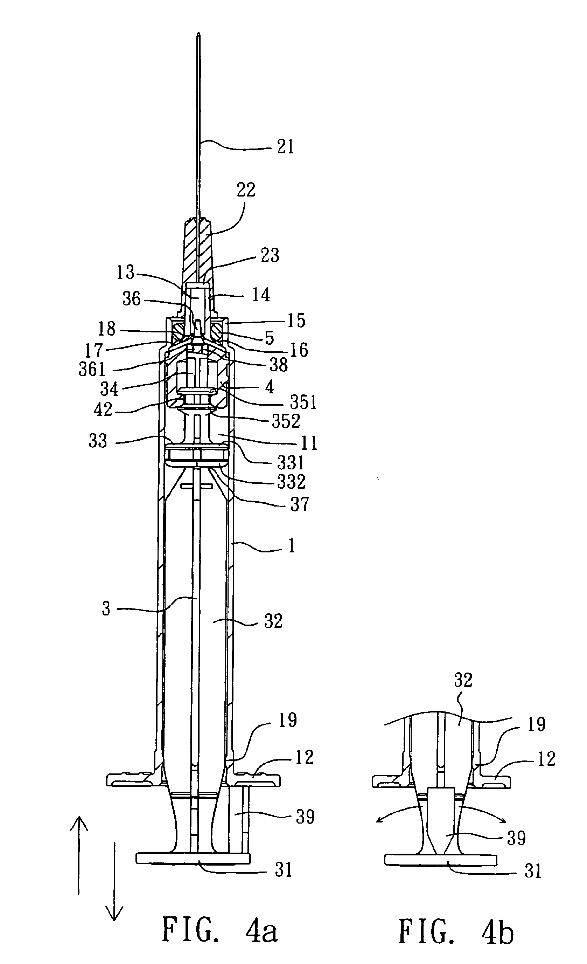

The plunger **3** comprises a radially ribbed shank **32,** a thumb rest **31** at the rear side of the shank **32,** a head **33** at the front side of the shank **32,** a front extension rod **34** axially forwardly extending from the center of the head **33,** a conical front tip **36,** a first neck **361** axially connected between the front end of the front extension rod **34** and the rear end of the conical front tip **36,** a stopper holder **35** formed of a parachute-like front skirt **351** and a parachute-like rear skirt **352** around the periphery of the front extension rod **34** for holding the stopper **4,** a second neck **37** axially connected between the front end of the shank **32** and the rear side of the head **33,** a sloping face **38** at the front end of the front extension rod **34,** and a protruding strip **39** forwardly extending from the thumb rest **31.** The protruding strip **39** has the reduced rear end connected to the thumb rest **31** (see FIG. 4b). Therefore, the protruding strip **39** can easily be biased and separated from the thumb rest **31** with the hand. The protruding strip **39** is stopped against the finger flange **12** of the barrel, preventing engagement of the front tip **36** into the annular retaining rib **18** of the barrel **11** accidentally during transportation of the safety hypodermic syringe.

The stopper **4** is made out of a flexible material, for example, rubber. The outer diameter of the stopper **4** fits the diameter of the fluid chamber **11** of the barrel **1.** The stopper **4** has a longitudinal open chamber **41** axially extending to the rear side, an inside annular flange **42** formed in the rear side of the longitudinal open chamber **41** and engaged in between the front skirt **351** and rear skirt **352** of the stopper holder **35** of the plunger **3,** and a front center through hole **43** forwardly extending from the longitudinal chamber **41** to the front side at the center for the passing of the front extension rod **34** of the plunger **3.**

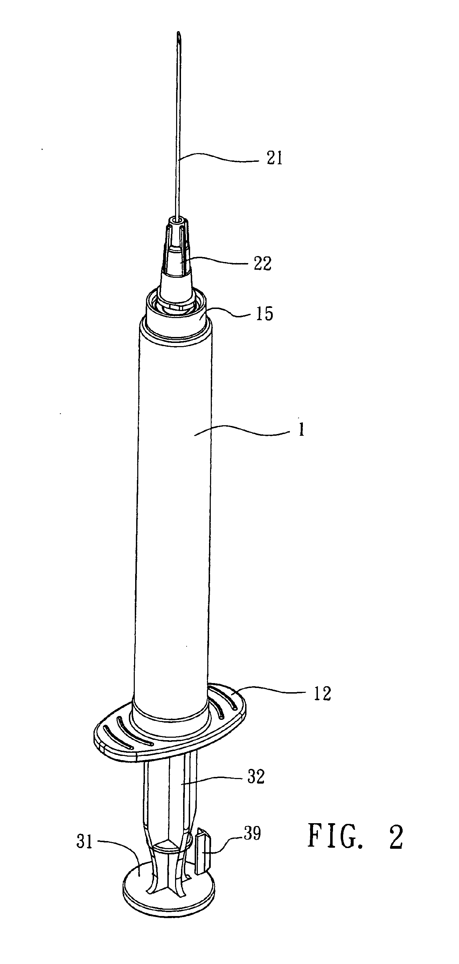

During the assembly process of the present invention, the stopper **4** is fastened to the front extension rod **34** of the plunger **3** to have the conical front tip **36** extend out of the front center through hole **43** and the front skirt **351** and rear skirt **352** of the stopper holder **35** of the plunger **3** be stopped at the front and rear sides of the inside annular flange **42** of the stopper **4,** and then the stopper **4** is inserted with the plunger **3** into the fluid chamber **11** of the barrel **1** from the rear side, and then the hub **21** of the needle assembly **2** is fastened to the tubular neck **14** of the barrel **1.** FIG. 2 shows the assembled status of the safety hypodermic syringe. FIG. 4a is a sectional view of FIG. 2.

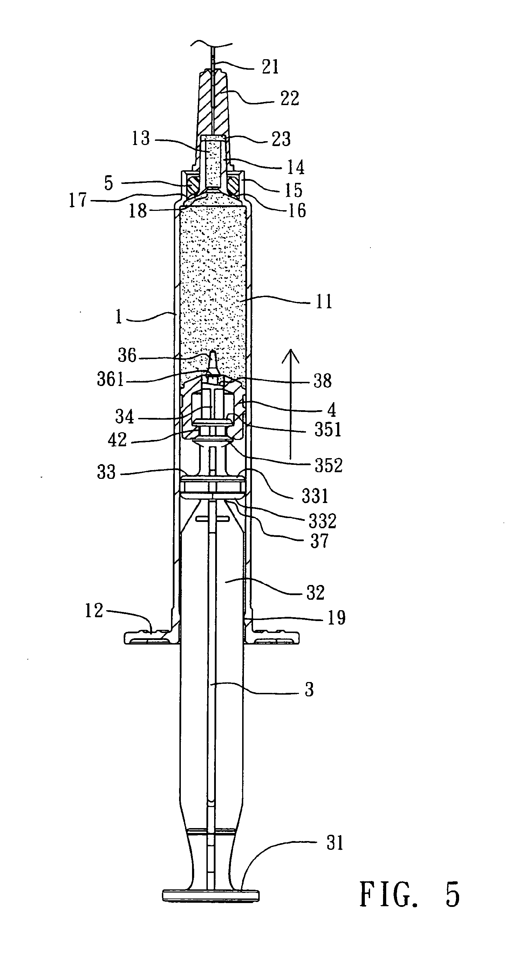

After the safety hypodermic syringe has sucked in the fluid medicine for injection, as shown in FIG. 5, push the plunger **3** to move the stopper **4** forwards along the fluid chamber **11** to squeeze the fluid medicine out of the tubular neck **14** and the needle assembly **2** into the patient's body.

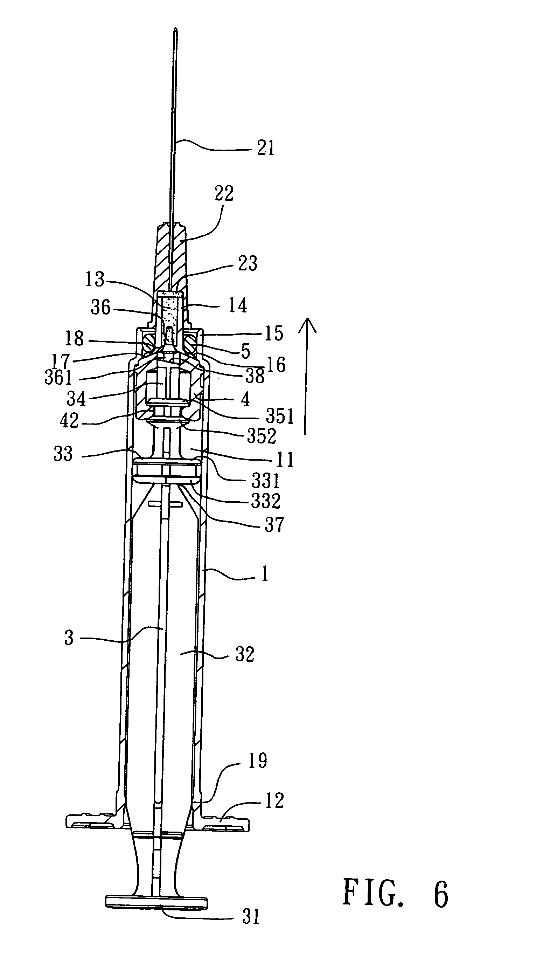

When the stopper **4** reached the front wall **16** of the barrel **1** as shown in FIG. 6, the conical front tip **36** is forced into the fluid passage **13** of the barrel **1,** preventing accumulation of residual fluid medicine in the barrel **1.**

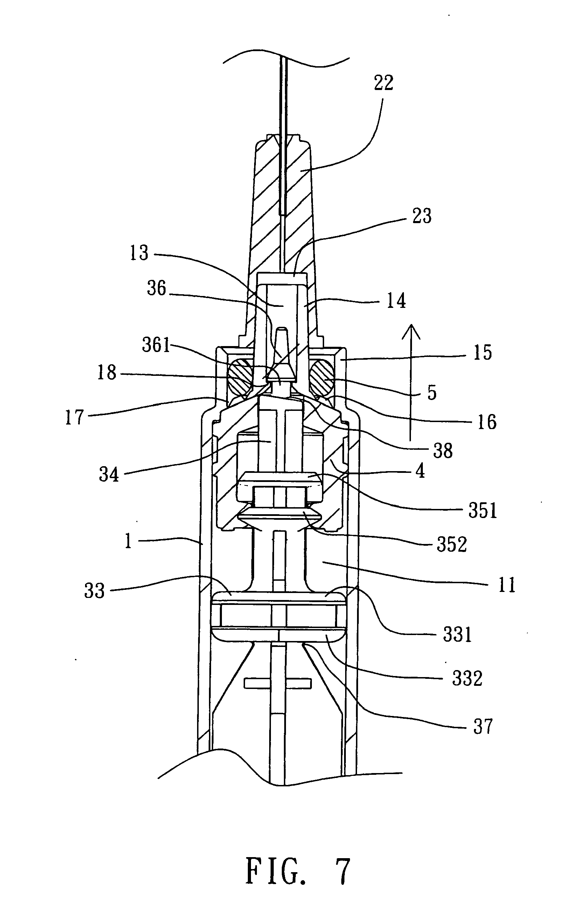

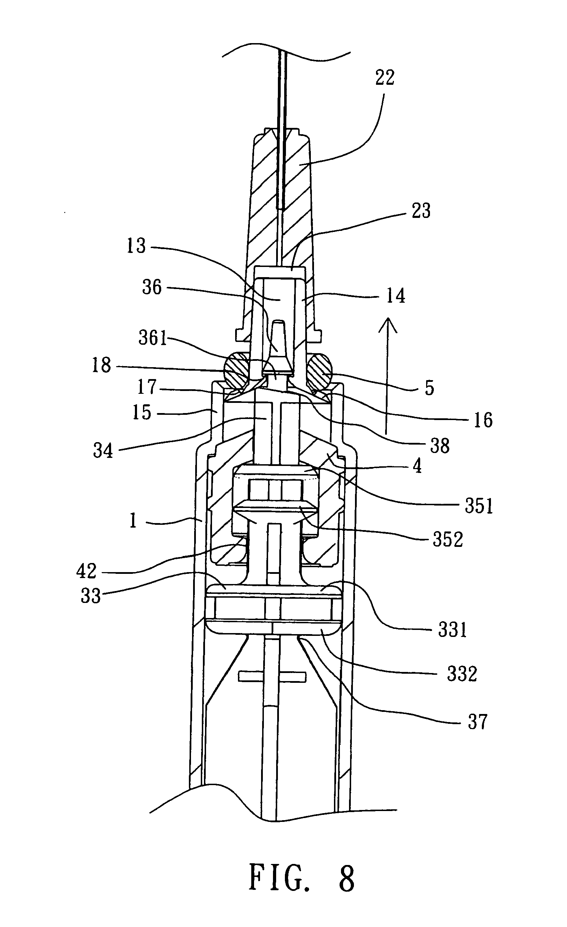

After the service of the safety hypodermic syringe as shown in FIG. 7, push the plunger **3** forwards again to force the first neck **361** into engagement with the annular retaining rib **18** inside the tubular neck **14** and simultaneously to move the front and rear skirts **351** and **352** of the stopper holder **35** into the longitudinal open chamber **41** as shown in FIG. 8. When continuously pushing the plunger **3** forwards to have the front skirt **351** be stopped against the inside annular flange **41** of the stopper **4,** the spiral tear groove **17** is forced to break, causing separation of the top wall **16** from the barrel **1** At this time, the broken barrel **1** becomes useless.

Thereafter, the plunger **3** is pulled backwards. At this time, the stopper **4,** the broken front wall **16,** the support ring **5,** the tubular neck **14** and the attached needle assembly **2** are carried with the plunger **3** into the inside of the fluid chamber **11** of the barrel **1** to have front flange **331** and rear flange **332** of the head **3** be respectively stopped at the front and rear sides of the inside annular stop flange **19** of the barrel **1.** At this time, the broken front wall **16** is moved along the sloping face **38** of the front extension rod **36** to bias the needle assembly **2** in the fluid chamber **11** of the barrel **1.**

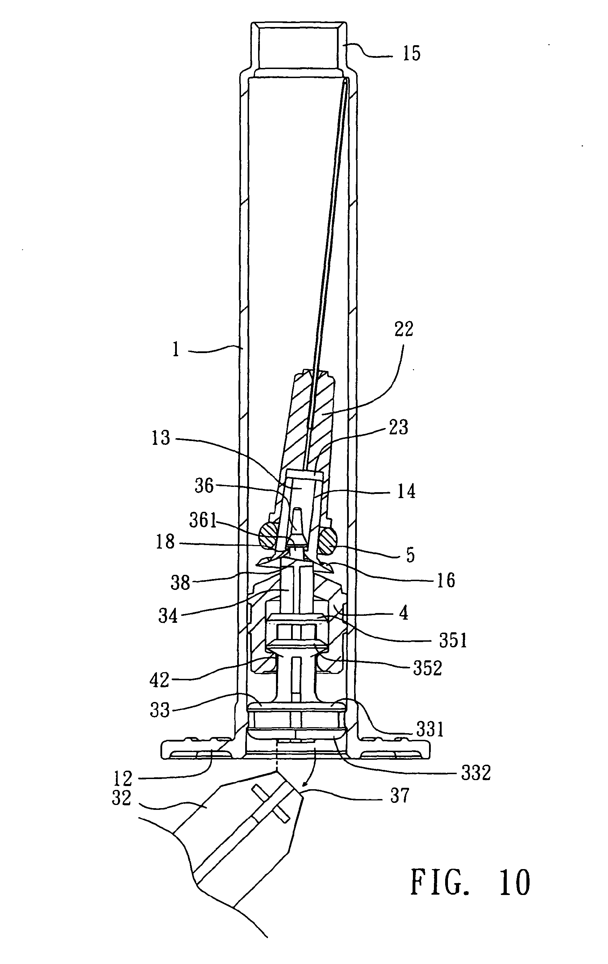

After the needle assembly **2** has been received inside the fluid chamber **11** of the barrel **1,** the user can bias the shank **32** against the inside wall of the barrel **1** to break the second neck **37,** as shown in FIG. 10, keeping the needle assembly **2** and a part of the plunger **3** and the stopper **4** inside the fluid chamber **11** of the barrel **1**.

As indicated above, the safety hypodermic syringe enables the needle assembly to be received inside the barrel after the service, preventing a reuse of the hypodermic syringe. The support ring is sleeved onto the tubular neck and stopped against the front wall of the barrel between the tubular neck and the race to support the front wall and to prevent breaking of the tear groove accidentally and also to prevent leakage of the contained liquid medicine out of the fluid chamber when the tear groove is broken accidentally. After the service of the safety hypodermic syringe, the user can push the plunger forwards to force the first neck into engagement with the annular retaining rib inside the tubular neck and simultaneously to move the front and rear skirts of the stopper holder into the longitudinal open chamber so that continuously pushing the plunger forwards can effectively break the spiral tear groove to separate the top wall from the barrel. The design of the present invention presents excessive residual liquid medicine in the barrel. The sloping face of the front extension rod guides backward movement of the needle assembly in an oblique manner so that the needle assembly can be positively received inside the barrel after the service of the safety hypodermic syringe. Further, the foolproof design of the protruding strip at the thumb rest prevents breaking of the spiral tear groove accidentally during transportation.

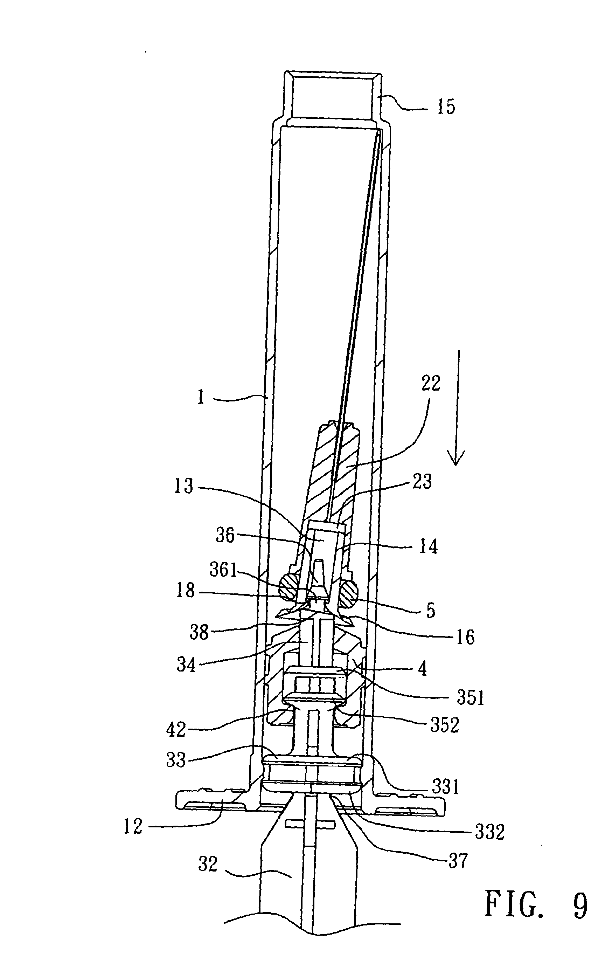

A prototype of safety hypodermic syringe has been constructed with the features of FIGS. 1-10. The safety hypodermic syringe functions smoothly to provide all the features discussed earlier.

Although a particular embodiment of the invention has been described in detail for purposes of illustration, various modifications and enhancements may be made without departing from the scope of the invention. Accordingly, the invention is not to be limited except as by the appended claims.

## Claims

1. A safety hypodermic syringe comprising:
a barrel (1), said barrel comprising a fluid chamber (11), a front wall (16), a tubular neck (14) forwardly and perpendicularly extending from said front wall, said tubular neck defining a fluid passage (13) in communication with said fluid chamber (11), a race (15) forwardly and perpendicularly extending from said front wall around said tubular neck (14), a spiral tear groove (17) formed on said front wall adjacent to said race, and annular retaining rib (18) extending around an inside wall of said tubular neck (14) adjacent to said front wall;
a needle assembly (2), said needle assembly comprising a hub (22) fitted onto said tubular neck of said barrel and a needle cannula (21) forwardly extending from said hub (22);
a plunger (3), said plunger comprising a head (33) at a front side of a shank (32) thereof, a front extension rod (34) axially forwardly extending from said head, a conical front tip (36), and a first neck (361) axially connected between said front extension rod and said conical front tip;
a flexible stopper (4) fitting the diameter of said fluid chamber (11) of said barrel (1), said stopper having a longitudinal open chamber (41) axially extending to a rear side thereof and a front center through hole (43) forwardly extending from said longitudinal chamber to a front side thereof for the passing of the front extension rod of said plunger; and
a flexible support ring (5) sleeved onto said tubular neck (14) and stopped against said front wall (16) of said barrel (1) between said tubular neck (14) and said race (15);
wherein when pushed said plunger (3) forwards after the service of the safety hypodermic syringe, said spiral tear groove (17) is broken to separate said front wall (16) from said barrel for enabling the broken front wall and said needle assembly (2) to be pulled with said plunger (3) to the inside of said barrel upon a return stroke of said plunger at this time.

2. The safety hypodermic syringe as claimed in claim 1, wherein said barrel has an inside annular stop flange (19) extending around an inside wall near a rear end thereof; said head of said plunger has a front flange (331) and a rear flange (332) for engagement with the inside annular stop flange of said barrel after a return stroke of said plunger after the service of the safety hypodermic syringe.

3. The safety hypodermic syringe as claimed in claim 1, wherein said plunger further has a rear neck (37) connected between said shank (32) and said head (33) for breaking to separate said shank from said head after engagement of the front flange and rear flange of said head with the inside annular stop flange of said barrel.

4. The safety hypodermic syringe as claimed in claim 1, wherein said support ring (5) is made out of an elastic material.

5. The safety hypodermic syringe as claimed in claim 1, wherein said shank of said plunger is radially ribbed, having a thumb rear at a rear end thereof.

6. The safety hypodermic syringe as claimed in claim 1, wherein said plunger has a stopper holder formed of a parachute-like front skirt (351) and a parachute-like rear skirt (352) around the periphery of said front extension rod (34) for holding said stopper; said stopper has an inside annular flange (42) formed in a rear side of said longitudinal chamber and engaged in between said front skit and rear skirt of said stopper holder of said plunger.

7. The safety hypodermic syringe as claimed in claim 1, wherein said front extension rod (34) of said plunger has a sloping face (38) adapted to guide backward movement of said broken front wall to the inside said barrel to bias said needle assembly in said fluid chamber of said barrel after a return stroke of said plunger after the service of the safety hypodermic syringe.

8. The safety hypodermic syringe as claimed in claim 1, wherein said plunger further comprises a thumb rest at a rear end of said shank and a protruding strip (39) forwardly extending from said thumb rest for stopping against a finger flange at a rear end of said barrel before the service of the safety hypodermic syringe, said protruding strip having a breakable reduced rear end connected to said thumb rest.

## Patentansprüche

1. Eine Sicherheitsinjektionsspritze, aufweisend:
ein Gehäuse (1), wobei das Gehäuse eine Fluidkammer (11), eine Stirnwand (16), einen rohrförmigen Hals (14), welcher sich von der Stirnwand nach vorne und senkrecht dazu erstreckt, wobei der rohrförmige Hals einen Fluidkanal (13) definiert, welcher in Verbindung ist mit der Fluidkammer (11), einen Ring (15), welcher sich von der Stirnwand nach vorne und senkrecht dazu erstreckt und zwar um den rohrförmigen Hals (14) herum, eine spiralförmige Abreissnut (17), welche benachbart zu dem Ring an der Stirnwand geformt ist, und eine ringförmige Halterippe (18) aufweist, welche sich benachbart zu der Stirnwand um die Innenwandung des rohrförmigen Halses (14) herum erstreckt,
eine Nadelvorrichtung (2), wobei die Nadelvorrichtung ein Basisteil (22), welches an dem rohrförmigen Hals des Gehäuses montiert ist, und eine Nadelkanüle (21) aufweist, welche sich von dem Basisteil (22) nach vorne erstreckt,
einen Kolben (3), wobei der Kolben einen Kopf (33) an einer Frontseite eines Schafts (32) davon, einen vorderen Fortsatzstab (34), welcher sich von dem Kopf axial nach vorne erstreckt, eine konische Frontspitze (36) und einen ersten Hals (361) aufweist, welcher axial zwischen dem vorderen Fortsatzstab und der konischen Frontspitze verbunden ist,
einen flexiblen Stopper (4), welcher in den Durchmesser der Fluidkammer (11) des Gehäuses (1) passt,
wobei der Stopper eine offene Längskammer (41), welche sich axial zu einer Hinterseite davon erstreckt, und ein vorderes zentrales Durchgangsloch (43) aufweist, welches sich von der Längskammer nach vorne zu einer Stirnseite davon erstreckt für den Durchtritt des vorderen Fortsatzstabes des Kolbens, und
einen flexiblen Stützring (5), welcher auf den rohrförmigen Hals (14) gestülpt ist und welcher zwischen dem rohrförmigen Hals (14) und dem Ring (15) an der Stirnwand (16) des Gehäuses (1) abgestützt ist,
wobei, wenn der Kolben (3) nach dem Betrieb der Sicherheitsinjektionsspritze nach vorne gedrückt wird, die spiralförmige Abreissnut (17) gebrochen wird, um die Stirnwand (16) von dem Gehäuse zu trennen, um zu ermöglichen, dass die abgebrochene Stirnwand und die Nadelvorrichtung (2) mit dem Kolben (3) in das Innere des Gehäuses gezogen werden auf einen Rückhub des Kolbens zu dieser Zeit.

2. Die Sicherheitsinjektionsspritze wie in Anspruch 1 beansprucht, wobei das Gehäuse einen inneren ringförmigen Stoppflansch (19) aufweist, welcher sich um eine Innenwandung herum nahe einem hinteren Ende davon erstreckt, wobei der Kopf des Kolbens einen vorderen Flansch (331) und einen hinteren Flansch (332) aufweist zum Eingreifen mit dem inneren ringförmigen Stoppflansch des Gehäuses nach einem Rückhub des Kolbens nach dem Betrieb der Sicherheitsinjektionsspritze.

3. Die Sicherheitsinjektionsspritze wie in Anspruch 1 beansprucht, wobei der Kolben ferner einen hinteren Hals (37), welcher zwischen dem Schaft (32) und dem Kopf (33) verbunden ist, zum Brechen aufweist, um nach dem Eingriff des vorderen Flansches und des hinteren Flansches des Kopfes mit dem inneren ringförmigen Stoppflansch des Gehäuses den Schaft von dem Kopf zu trennen.

4. Die Sicherheitsinjektionsspritze wie in Anspruch 1 beansprucht, wobei der Stützring (5) aus einem elastischen Material hergestellt ist.

5. Die Sicherheitsinjektionsspritze wie in Anspruch 1 beansprucht, wobei der Schaft des Kolbens mit radialen Rippen versehen ist und an einem hinteren Ende davon ein Daumenhinterteil aufweist.

6. Die Sicherheitsinjektionsspritze wie in Anspruch 1 beansprucht, wobei der Kolben einen Stopperhalter zum Halten des Stoppers aufweist, welcher geformt ist aus einem schirmartigen vorderen Ringbund (351) und einem schirmartigen hinteren Ringbund (352) um den Umfang des vorderen Fortsatzstabes (34) herum, wobei der Stopper einen inneren ringförmigen Flansch (42) aufweist, welcher in einer Hinterseite von der Längskammer geformt ist und mit dem vorderen Ringbund und dem hinteren Ringbund des Stopperhalters des Kolbens im Eingriff steht.

7. Die Sicherheitsinjektionsspritze wie in Anspruch 1 beansprucht, wobei der vordere Fortsatzstab (34) des Kolbens eine schräge Fläche (38) aufweist, welche angepasst ist, um die Rückwärtsbewegung der abgebrochenen Stirnwand in das Innere des Gehäuses zu führen, um die Stellung der Nadelvorrichtung in der Fluidkammer des Gehäuses nach einem Rückhub des Kolbens nach dem Betrieb der Sicherheitsinjektionsspritze zu beeinflussen.

8. Die Sicherheitsinjektionsspritze wie in Anspruch 1 beansprucht, wobei der Kolben ferner aufweist eine Daumenstütze an einem hinteren Ende des Schafts und einen vorspringenden Streifen (39), welcher von der Daumenstütze nach vorne vorspringt, zum Anschlagen an einen Fingerflansch an einem hinteren Ende des Gehäuses vor dem Betrieb der Sicherheitsinjektionsspritze, wobei der vorspringende Streifen ein abbrechbares reduziertes hinteres Ende aufweist, welches mit der Daumenstütze verbunden ist.

## Revendications

1. Seringue hypodermique de sécurité comprenant:
- un cylindre (1), ledit cylindre comprenant une chambre à fluide (11), une paroi avant (16), un col tubulaire (14) s'étendant vers l'avant et perpendiculairement à partir de ladite paroi avant, ledit col tubulaire définissant un passage de fluide (13) en communication avec ladite chambre à fluide (11), un chemin (15) s'étendant vers l'avant et perpendiculairement à partir de ladite paroi avant autour dudit col tubulaire (14), une rainure de rupture en spirale (17) formée sur ladite paroi avant, adjacente audit chemin, et une nervure de retenue annulaire (18) s'étendant autour d'une paroi intérieure dudit col tubulaire (14) adjacente à ladite paroi avant ;
- un ensemble aiguille (2), ledit ensemble aiguille comprenant un moyeu (22) adapté sur ledit col tubulaire dudit cylindre et une canule d'aiguille (21) s'étendant vers l'avant à partir dudit moyeu (22);
- un piston (3), ledit piston comprenant une tête (33) sur un côté avant d'un corps (32) de celui-ci, une tige d'extension avant (34) s'étendant axialement vers l'avant à partir de ladite tête, une pointe avant conique (36), et un premier col (361) axialement relié entre ladite tige d'extension avant et ladite pointe avant conique ;
- un bouchon souple (4), s'adaptant au diamètre de ladite chambre à fluide (11) dudit cylindre (1), ledit bouchon ayant une chambre ouverte longitudinale (41) s'étendant axialement vers un côté arrière de celui-ci et un trou traversant central avant (43) s'étendant vers l'avant de ladite chambre longitudinale à un côté avant de celui-ci pour le passage de la tige d'extension avant dudit piston ; et
- un anneau de support souple (5) placé sur ledit col tubulaire (14) et arrêté contre ladite paroi avant (16) dudit cylindre (1) entre ledit col tubulaire (14) et ledit chemin (15) ;
dans laquelle, lorsque ledit piston (3) est poussé vers l'avant après l'utilisation de la seringue hypodermique de sécurité, ladite rainure de rupture en spirale (17) est rompue pour séparer ladite paroi avant (16) dudit cylindre pour permettre à la paroi avant séparée par rupture et audit ensemble aiguille (2) d'être tirés avec ledit piston (3) à l'intérieur dudit corps lors d'une course de retour dudit piston à ce moment.

2. Seringue hypodermique de sécurité selon la revendication 1, dans laquelle ledit cylindre a une collerette d'arrêt annulaire intérieure (19) s'étendant autour d'une paroi intérieure proche d'une extrémité arrière de celui-ci ; ladite tête dudit piston a une collerette avant (331) et une collerette arrière (332) pour un engagement avec la collerette d'arrêt annulaire intérieure dudit cylindre après une course de retour dudit piston après l'utilisation de la seringue hypodermique de sécurité.

3. Seringue hypodermique de sécurité selon la revendication 1, dans laquelle ledit piston a en outre un col arrière (37) relié entre ledit corps (32) et ladite tête (33) pour séparer par rupture ledit corps de ladite tête après l'engagement de la collerette avant et de la collerette arrière de ladite tête avec la collerette d'arrêt annulaire intérieure dudit cylindre.

4. Seringue hypodermique de sécurité selon la revendication 1, dans laquelle ledit anneau de support (5) est fait en matériau élastique.

5. Seringue hypodermique de sécurité selon la revendication 1, dans laquelle ledit corps dudit piston est radialement nervuré, ayant un arrière pour pouce à une extrémité arrière de celui-ci.

6. Seringue hypodermique de sécurité selon la revendication 1, dans laquelle ledit piston a un porte-bouchon formé d'une jupe avant de type parachute (351) et d'une jupe arrière de type parachute (352) autour de la périphérie de ladite tige d'extension avant (34) pour maintenir ledit bouchon ; ledit bouchon a une collerette annulaire intérieure (42) formée sur un côté arrière de ladite chambre longitudinale et engagée entre lesdites jupes avant et arrière dudit porte-bouchon dudit piston.

7. Seringue hypodermique de sécurité selon la revendication 1, dans laquelle ladite tige d'extension avant (34) dudit piston a une face inclinée (38) apte à guider un mouvement vers l'arrière de ladite paroi avant séparée par rupture vers l'intérieur dudit cylindre pour solliciter ledit ensemble aiguille dans ladite chambre à fluide dudit cylindre après une course de retour dudit piston après l'utilisation de la seringue hypodermique de sécurité.

8. Seringue hypodermique de sécurité selon la revendication 1, dans laquelle ledit piston comprend un repose-pouce à une extrémité arrière dudit corps et une bande saillante (39) s'étendant vers l'avant à partir dudit repose-pouce pour s'arrêter contre une collerette à doigt à une extrémité arrière dudit cylindre avant l'utilisation de la seringue hypodermique de sécurité, ladite bande saillante ayant une extrémité arrière réduite séparable par rupture, reliée audit repose-pouce.
